# EUROPEAN PATENT APPLICATION

(11) **EP 4 414 447 A1**
(43) Date of publication of application: **14.08.2024**
(21) Application number: 22878639.8
(22) Date of filing: 07.10.2022
(51) Int. Cl.: C12N 1/21, C12P 19/28, C12N 15/53, C12N 15/54, C12N 15/61

(54) **MICROORGANISM HAVING ABILITY TO PRODUCE N-ACETYLNEURAMINIC ACID AND/OR N-ACETYLNEURAMINIC ACID-CONTAINING CARBOHYDRATE AND METHOD FOR PRODUCING N-ACETYLNEURAMINIC ACID AND/OR N-ACETYLNEURAMINIC ACID-CONTAINING CARBOHYDRATE USING SAID MICROORGANISM**

(30) Priority: 08.10.2021 JP 2021166476
(71) Applicant: Kyowa Hakko Bio Co., Ltd., Tokyo 100-8185 (JP)
(72) Inventor: NAOE, Taiki, Tokyo 100-8185 (JP); UJIHARA, Tetsuro, Tokyo 100-8185 (JP)
(74) Representative: Vossius & Partner Patentanwälte Rechtsanwälte mbB
(86) International application number: PCT/JP2022/037736
(87) International publication number: WO 2023/058772

(57) **Abstract**

An object of the present invention is to provide a microorganism having an ability to produce NeuAc and/or a NeuAc-containing carbohydrate, and a method for producing NeuAc and/or the NeuAc-containing carbohydrate more efficiently by the microorganism. The present invention relates to a microorganism which has an ability to produce NeuAc and/or a NeuAc-containing carbohydrate and whose enterobacterial common antigen (ECA) biosynthesis pathway is blocked, and a method for producing NeuAc and/or the NeuAc-containing carbohydrate using the microorganism.

## Description

### TECHNICAL FIELD

The present invention relates to a microorganism having an ability to produce a N-acetylneuraminic acid (hereinafter, also abbreviated as NeuAc) and/or a NeuAc-containing carbohydrate, and a method for efficiently producing NeuAc and/or the NeuAc-containing carbohydrate using the microorganism.

### BACKGROUND ART

N-acetylneuraminic acid (hereinafter, referred to as NeuAc) is a kind of amino sugar contained in a human body, and is generally known to be localized as sialic acid at a non-reducing terminal of a glycoprotein or a glycolipid. NeuAc is contained in gangliosides and glycolipids in the brain in a large amount, and it is suggested that NeuAc is associated with the development and cognitive functions of the brain (Non-Patent Literature 1).

As a method for producing NeuAc, a method for extraction from natural resources such as a nest of swallows and egg yolks, a method for hydrolysis of a polymer, and the like have been used for a long time, but the complexity and low yield of the production method are problems (Non-Patent Literature 2).

On the other hand, as a more efficient method for producing NeuAc, a production method using an enzyme and a production method based on direct fermentation using a microorganism have been developed (Non-Patent Literature 2).

Examples of known production methods using an enzyme include a method for producing NeuAc by a NeuAc aldolase reaction using pyruvic acid and N-acetylmannosamine (hereinafter, referred to as ManNAc) as substrates (Non-Patent Literatures 3 and 4), a method for producing NeuAc by a NeuAc aldolase reaction under an alkali condition using pyruvic acid and N-acetylglucosamine (hereinafter referred to as GlcNAc) as substrates (Patent Literature 1), a method for producing NeuAc by a reaction of NeuAc aldolase and N-acetylglucosamine-2-epimerase (hereinafter referred to as GlcNAc epimerase) using pyruvic acid and GlcNAc as substrates (Non-Patent Literatures 5 and 6), and a method for producing NeuAc by a NeuAc synthetase reaction using phosphoenolpyruvate (hereinafter, referred to as PEP) and ManNAc as substrates (Patent Literature 2 and Non-Patent Literature 7). However, all the methods for producing NeuAc using the above enzymes have the problem of complicated operations.

As the production method based on direct fermentation using a microorganism, for example, Patent Literature 4 discloses a method in which a neuC gene encoding UDP-GlcNAc epimerase derived from Campylobacter jejuni and a neuB gene encoding sialic acid synthase are introduced into Escherichia coli, and GlcNAc is transformed into NeuAc via ManNAc (the pathway is called the NeuC pathway). Patent Literature 5 discloses a method for producing NeuAc from GlcNAc via ManNAc by producing GlcNAc from glucosamine 6-phosphate by the introduction of a GNA1 gene encoding glucosamine acetyltransferase derived from Saccharomyces cerevisiae, and further introducing an slr1975 gene encoding N-acetylmannosamine epimerase derived from cyanobacterium Synechocystis sp.PCC6803 disclosed in Patent Literature 3 and a neuB gene encoding a sialic acid synthase derived from Rhodobacter (the pathway is referred to as GNA1 pathway).

Unlike the NeuC pathway, the GNA1 pathway does not pass through activated sugar nucleotides such as UDP-GlcNAc, and thus is considered to be advantageous for production. However, there are problems in the production efficiency and manufacturing cost of NeuAc currently as described in Non-Patent Literature 8.

The Escherichia coli has a wecB gene (also known as yifF gene, nfrC gene, or rffE gene) as a gene encoding UDP-GlcNAc epimerase (WecB) for transformation from UDP-GlcNAc to UDP-ManNAc (Non-Patent Literature 9). As described above, UDP-GlcNAc epimerase is an essential enzyme in the NeuC pathway. However, it is shown that WecB functions complementarily in the production of NeuAc even in the GNA1 pathway (Non-Patent Literature 8).

In addition, WecB is known to function in Escherichia coli to biosynthesize enterobacterial common antigen (hereinafter referred to as ECA) (Non-Patent Literature 9). The ECA biosynthesis pathway is well known and WecA, WecC, and the like, in addition to WecB, are known to be involved in the ECA biosynthesis pathway (Non-Patent Literatures 9 and 10).

However, a relationship between the ECA biosynthesis pathway and the production of NeuAc or the NeuAc-containing carbohydrate is not known, and an influence on the production of NeuAc or the NeuAc-containing carbohydrate when a protein related to ECA biosynthesis such as WecB is lost is not known.

### CITATION LIST

### PATENT LITERATURE

Patent Literature 1: US5,665,574B
Patent Literature 2: JPH10-4961
Patent Literature 3: WO2015/037698
Patent Literature 4: WO2008/040717
Patent Literature 5: WO2012/112777
Patent Literature 6: WO2004/003175
Patent Literature 7: WO2008/097366

### NON-PATENT LITERATURE

Non-Patent Literature 1: Annu. Rev. Nutr. (2009), Vol. 29, p.177-222
Non-Patent Literature 2: Biotechnology Advances (2021), Vol.46, 107678
Non-Patent Literature 3: J. Am. Chem. Soc. (1988), Vol. 110, p.6481-6486
Non-Patent Literature 4: J. Am. Chem. Soc. (1988), Vol. 110, p.7159-7163
Non-Patent Literature 5: Angew. Chem. Int. Ed. Eng. (1991), Vol. 30, p.827-828
Non-Patent Literature 6: Carbohydrate Research (1998), Vol. 306, p.575-578
Non-Patent Literature 7: Glycobiology (1997), Vol. 7, p.697-701
Non-Patent Literature 8: Metab. Eng. (2012), Vol. 14, p.623-629
Non-Patent Literature 9: FEMS Microbiol. Rev. (1988), Vol. 54, p.195-222
Non-Patent Literature 10: J Gen Appl Microbiol (2020), Vol. 66, p.169-174

### SUMMARY OF INVENTION

### TECHNICAL PROBLEM

An object of the present invention is to provide a microorganism having an ability to produce NeuAc and/or a NeuAc-containing carbohydrate, and a method for producing NeuAc and/or the NeuAc-containing carbohydrate more efficiently by the microorganism.

### SOLUTION TO PROBLEM

The present inventors have found that NeuAc and/or a NeuAc-containing carbohydrate can be produced more efficiently than in the related art by using a microorganism which has an ability to produce NeuAc and/or a NeuAc-containing carbohydrate and whose ECA biosynthesis pathway is blocked, and have completed the present invention.

That is, the present invention is as follows.
1. A microorganism which has an ability to produce at least one of N-acetylneuraminic acid and a N-acetylneuraminic acid-containing carbohydrate and whose enterobacterial common antigen (ECA) biosynthesis pathway is blocked.
2. The microorganism according to the above 1, in which an activity of at least one protein selected from the following [1] to [3] is lost,
   [1] a protein consisting of an amino acid sequence represented by SEQ ID NO: 2 or a homologous sequence thereof and having a UDP-N-acetylglucosamine-undecaprenyl phosphate N-acetylglucosamine phosphotransferase (WecA) activity;
   [2] a protein consisting of an amino acid sequence represented by SEQ ID NO: 4 or a homologous sequence thereof and having a UDP-N-acetylglucosamine 2-epimerase (WecB) activity; and
   [3] a protein consisting of an amino acid sequence represented by SEQ ID NO: 6 or a homologous sequence thereof and having UDP-N-acetylmannosamine dehydrogenase (WecC) activity.
3. The microorganism according to the above 1 or 2, in which the microorganism is Escherichia coli.
4. A method for producing at least one of N-acetylneuraminic acid and a N-acetylneuraminic acid-containing carbohydrate, the method including culturing the microorganism according to any one of the above 1 to 3 in a culture medium to produce at least one of N-acetylneuraminic acid and the N-acetylneuraminic acid-containing carbohydrate.

### ADVANTAGEOUS EFFECTS OF INVENTION

A microorganism according to the present invention is a microorganism which has an ability to produce NeuAc and/or a NeuAc-containing carbohydrate and whose ECA biosynthesis pathway is blocked, whereby exhibiting an excellent ability to produce NeuAc and/or a NeuAc-containing carbohydrate. By using the microorganism according to the present invention, a production amount of NeuAc and/or a NeuAc-containing carbohydrate can be increased and impurities can be reduced as compared with the related art, and NeuAc and/or the NeuAc-containing carbohydrate can be efficiently produced.

### DESCRIPTION OF EMBODIMENTS

### 1. Microorganism according to present invention and method for constructing same

The microorganism according to the present invention is a microorganism which has an ability to produce NeuAc and/or a NeuAc-containing carbohydrate and whose ECA biosynthesis pathway is blocked, whereby having an improved production efficiency of NeuAc and/or the NeuAc-containing carbohydrate.

Examples of the microorganism according to the present invention include the following microorganisms (I) and (II).
(I) A microorganism obtained by using a microorganism that originally has the ability to produce NeuAc and/or a NeuAc-containing carbohydrate as a parent strain and blocking the ECA biosynthesis pathway of the parent strain
(II) A microorganism in which an ability to produce NeuAc and/or a NeuAc-containing carbohydrate is artificially imparted or enhanced using a microorganism whose ECA biosynthesis pathway is blocked as a parent strain.

In the present invention, the parent strain refers to an original strain to be subjected to genetic modification, transformation, and the like. The original strain to be subjected to transformation by genetic transfer is also referred to as a host strain.

The parent strain is any microorganism. The parent strain is preferably a prokaryote or a yeast strain, is more preferably a prokaryote belonging to the genus Escherichia, the genus Serratia, the genus Bacillus, the genus Brevibacterium, the genus Corynebacterium, the genus Microbacterium, the genus Pseudomonas, or the like, or a yeast strain belonging to the genus Saccharomyces, the genus Schizosaccharomyces, the genus Kluyveromyces, the genus Trichosporon, the genus Siwaniomyces, the genus Pichia, the genus Candida, or the like, and is most preferably a prokaryote such as Escherichia coli BL21 codon plus, Escherichia coli XL1-Blue, and Escherichia coli XL2-Blue (all manufactured by Agilent Technologies, Inc.), Escherichia coli BL21 (DE3) pLysS (manufactured by Merck Millipore Inc.), Escherichia coli DH5α, Escherichia coli HST08 Premium, Escherichia coli HST02, Escherichia coli HST04 dam⁻/dcm⁻, Escherichia coli JM109, Escherichia coli HB101, Escherichia coli CJ236, Escherichia coli BMH71-18 mutS, Escherichia coli MV1184, and Escherichia coli TH2 (all manufactured by Takara Bio Inc.), Escherichia coli W, Escherichia coli JM101, Escherichia coli W3110, Escherichia coli MG1655, Escherichia coli DH1, Escherichia coli MC1000, Escherichia coli W1485, Escherichia coli MP347, Escherichia coli NM522, Escherichia coli ATCC9637, Escherichia coli BW25113, Serratia ficaria, Serratia fonticola, Serratia liquefaciens, Serratia marcescens, Bacillus subtilis, Bacillus amyloliquefaciens, Brevibacterium immariophilum ATCC 14068, Brevibacterium saccharolyticum ATCC 14066, Corynebacterium ammoniagenes, Corynebacterium glutamicum ATCC13032, Corynebacterium glutamicum ATCC14067, Corynebacterium glutamicum ATCC13869, Corynebacterium acetoacidophilum ATCC13870, Microbacterium ammoniaphilumATCC15354], or Pseudomonas sp. D-0110, or a yeast strain such as Saccharomyces cerevisiae, Schizosaccharomyces pombe, Kluyveromyces lactis, Trichosporon pullulans, Schwanniomyces alluvius, Pichia pastoris, or Candida utilis.

### 1-1. Microorganism having ability to produce NeuAc and/or NeuAc-containing carbohydrate

In the specification, the microorganism having the ability to produce NeuAc and/or a NeuAc-containing carbohydrate refers to a microorganism having an ability to biosynthesize NeuAc and/or a NeuAc-containing carbohydrate when the microorganism is cultured in a culture medium.

As the microorganism having the ability to produce NeuAc and/or a NeuAc-containing carbohydrate, a breeding strain in which an ability to produce NeuAc and/or a NeuAc-containing carbohydrate is artificially imparted or enhanced can also be preferably used.

The production of NeuAc and/or a NeuAc-containing carbohydrate by the microorganism can be confirmed by, for example, culturing the microorganism in a culture medium and detecting NeuAc and/or the NeuAc-containing carbohydrate accumulated in the culture product using HPLC or a carbohydrate analyzer described below.

Examples of a method for artificially imparting or enhancing the ability to produce NeuAc and/or a NeuAc-containing carbohydrate include well-known methods such as the following (a) to (d). These methods can be used alone or in combination.
(a) A method for enhancing expression of at least one enzyme associated with a biosynthesis pathway for producing NeuAc and/or a NeuAc-containing carbohydrate from a saccharide
(b) A method for increasing the number of copies of at least one gene associated with a biosynthesis pathway for producing NeuAc and/or a NeuAc-containing carbohydrate from a saccharide
(c) A method for alleviating or releasing at least one mechanism for regulating a biosynthesis pathway for producing NeuAc and/or a NeuAc-containing carbohydrate from a saccharide
(d) A method for weakening or blocking at least one metabolic pathway branching off from a biosynthesis pathway for producing NeuAc and/or a NeuAc-containing carbohydrate from a saccharide to a metabolic product other than a target substance

In order to artificially enhance the ability to produce the NeuAc-containing carbohydrate, expression of at least one enzyme associated with assimilation or decomposition of the metabolic product other than the target substance or a compound derived from a culture medium component may be enhanced. For example, a method for artificially imparting or enhancing a protein associated with uptake of maltose and isomaltose and/or a protein associated with the decomposition of maltose for the purpose of assimilation of maltose or isomaltose derived from a culture medium component is exemplified. Specific examples of the method include a method described in WO2009/088049.

Examples of the protein associated with the uptake of maltose and isomaltose include well-known proteins such as PTS maltose transporter subunit IIBC MalP derived from Bacillus subtilis strain 168. Examples of the protein associated with the decomposition of maltose include well-known proteins such as maltose-6-phosphate glucosidase MalA derived from Bacillus subtilis strain 168.

In order to artificially enhance the ability to produce NeuAc and/or a NeuAc-containing carbohydrate, the consumption of phosphoenolpyruvate necessary for the production of NeuAc may be reduced. For example, for the purpose of reducing the consumption of phosphoenolpyruvate necessary for the production of NeuAc, at least one protein associated with conversion from pyruvic acid to oxaloacetic acid may be artificially imparted or enhanced. Specific examples of the method include a method described in Vemuri GN et. al. Biotechnol Bioeng 2005, 90: 64-76.

Examples of the protein associated with conversion from the above pyruvic acid to oxaloacetic acid include well-known proteins such as pyruvate carboxylase Pyc derived from Corynebacterium glumicum strain ATCC 13032.

### <Microorganism 1 in which ability to produce Neuac and/or Neuac-containing carbohydrate is artificially imparted or enhanced>

Among the microorganisms in which an ability to produce NeuAc is artificially imparted or enhanced, specific examples of microorganisms obtained by the method (a) or (b) include microorganisms in which an ability to produce at least one protein selected from the following [1] to [3] is artificially imparted or enhanced.
[1] NeuAc synthase
[2] GlcNAc epimerase
[3] N-acetylglucosamine-6-phosphate-N-acetyltransferase

Among the microorganisms in which the ability to produce NeuAc-containing carbohydrate is artificially imparted or enhanced, specific examples of the microorganisms obtained by the above method (a) or (b) include microorganisms in which an ability to produce at least one protein selected from the following [1] to [5] is imparted or enhanced.
[1] NeuAc synthase
[2] GlcNAc epimerase
[3] N-acetylglucosamine-6-phosphate-N-acetyltransferase
[4] CMP-NeuAc synthase
[5] Sialyltransferase

The microorganism for producing the above proteins can be prepared by, for example, introducing, into a parent strain, a recombinant DNA obtained by inserting a DNA fragment encoding the above protein into downstream of a promoter of an appropriate expression vector. When the recombinant DNA is a DNA that can be incorporated into the chromosomal DNA of the parent strain, a promoter may not be contained.

When a prokaryote such as bacteria is used as a parent strain, the recombinant DNA is preferably a recombinant DNA composed of a promoter, a ribosome binding sequence, a DNA containing a target gene, and a transcription termination sequence. A gene for regulating the promoter may be contained.

It is preferable to use a plasmid in which a distance between a Shine-Dalgarno sequence, which is a ribosome binding sequence, and an initiation codon is adjusted to an appropriate distance (for example, 6 to 18 bases). In the recombinant DNA, a transcription termination sequence is not necessarily required for expression of the DNA, but it is preferable to place the transcription termination sequence immediately after a structural gene.

The expression vector is not particularly limited as long as it is an appropriate nucleic acid molecule for introducing the target DNA into a host and causing the target DNA to be amplified and expressed. For example, not only plasmids, but also, for example, artificial chromosomes, vectors using transposons, and cosmids may be used.

When a microorganism belonging to the genus Escherichia is used as the parent strain, examples of the expression vector include pColdI, pSTV28, pSTV29, pUC118 (all manufactured by Takara Bio Inc.), pMW119 (manufactured by Nippon Gene Co., ltd.), pET21a, pCOLADuet-1, pCDFDuet-1, pCDF-1b, pRSF-1b (all manufactured by Merck Millipore Inc.), pMAL-c5x (manufactured by New England Biolabs), pGEX-4T-1, pTrc99A (both manufactured by GE Healthcare Bioscience), pTrcHis, pSE280 (both manufactured by Thermo Fisher Scientific K.K.), pGEMEX-1 (manufactured by Promega Corporation), pQE-30, pQE80L (both manufactured by Qiagen), pET-3, pBluescriptII SK(+), pBluescriptII KS(-) (all manufactured by Agilent Technologies, Inc.), pKYP10 (JPS58-110600A), pKYP200 [Agric. Biol. Chem., 48, 669 (1984)], pLSA1 [Agric. Biol. Chem., 53, 277 (1989)], pGEL1 [Proc. Natl. Acad. Sci., USA, 82, 4306 (1985)], pBluescript II SK (+), pBluescript II KS (-) (manufactured by Stratagene Corporation), pTrS30 [prepared from Escherichia coli JM109/pTrS30 (FERM BP-5407)], pTrS32 [prepared from Escherichia coli JM109/pTrS32 (FERM BP-5408)], pTK31 [APPLIED AND ENVIRONMENTAL MICROBIOLOGY, 2007, Vol.73, No.20, p6378-6385], pPAC31 (WO98/12343), pUC19 [Gene, 33, 103 (1985)], pPA1 (JPS63-233798A), and pKD46 [Proc. Natl. Acad. Sci., USA, 97, 6640-6645 (2000)].

The promoter in the case of using the above expression vector may be any promoter as long as it functions in cells of microorganisms belonging to the genus Escherichia, and for example, a promoter of a gene associated with amino acid biosynthesis, such as a trp promoter or an ilv promoter, and a promoter derived from, for example, an Escherichia coli or a phage, such as a uspA promoter, a lac promoter, a PL promoter, a PR promoter, or a PSE promoter can be used. A promoter that is artificially modified in design, such as a promoter with two trp promoters in series, a tac promoter, a trc promoter, a lacT7 promoter, or a letI promoter can also be used.

When a coryneform bacterium is used as the parent strain, examples of the expression vector include pCG1 (JPS57-134500A), pCG2 (JPS58-35197A), pCG4 (JPS57-183799A), pCG11 (JPS57-134500A), pCG116, pCE54, and pCB101 (all JPS58-105999A), pCE51, pCE52, and pCE53 [all Molecular and General Genetics, 196, 175, (1984)].

The promoter in the case of using the above expression vector may be any promoter as long as it functions in cells of the coryneform bacterium, and examples thereof include a P54-6 promoter [Appl. Microbiol. Biotechnol., 53, p674-679, (2000)].

When a yeast strain is used as the parent strain, examples of the expression vector include YEp13 (ATCC37115), YEp24 (ATCC37051), YCp50 (ATCC37419), pHS19, and pHS15.

The promoter in the case of using the above expression vector may be any promoter as long as it functions in cells of the yeast strain, and examples thereof include a PHOS promoter, a PGK promoter, a GAP promoter, an ADH promoter, a gal1 promoter, a gal10 promoter, a heat shock polypeptide promoter, an MFα1 promoter, and a CUP1 promoter.

Here, the expression level of the protein encoded by the DNA can also be improved by substituting a base such that the nucleotide sequence of the DNA is an optimal codon for expression in a parent strain. Information on the frequency of codon use in the parent strain used in the production method according to the present invention can be obtained through a public database.

Examples of a method for introducing a recombinant DNA into a host strain as an autonomously replicable plasmid include a method using calcium ions [Proc. Natl. Acad. Sci., USA, 69, 2110 (1972)], a protoplast method (JPS63-248394A), and an electroporation method [Nucleic Acids Res., 16, 6127 (1988)].

Examples of the method for incorporating a recombinant DNA into a chromosome of a parent strain include a homologous recombination method. Examples of the homologous recombination method include a method using a plasmid for homologous recombination that can be prepared by linking with a plasmid DNA having a drug resistance gene that cannot autonomously replicate in a host cell to be introduced. Examples of the method using homologous recombination frequently used in Escherichia coli include a method of introducing a recombinant DNA using a homologous recombination system of a lambda phage [Proc. Natl. Acad. Sci. USA, 97, 6641-6645 (2000)].

Further, by using a selection method based on the fact that Escherichia coli becomes sucrose-sensitive due to a Bacillus subtilis levansucrase incorporated on the chromosome together with a recombinant DNA, or a selection method based on the fact that Escherichia coli becomes streptomycin-sensitive by incorporating a wild-type rpsL gene into Escherichia coli comprising a mutant rpsL gene for streptomycin resistance [Mol. Microbiol., 55, 137 (2005), Biosci. Biotechnol. Biochem., 71, 2905 (2007)], a microorganism with a target region on a chromosomal DNA of the parent strain substituted with the recombinant DNA can be obtained. Here, the chromosomal region that causes introduction is not particularly limited, and is preferably an unessential genetic region or a non-genetic region upstream of the unessential genetic region.

DNAs encoding a protein selected from the group consisting of [1] NeuAc synthase, [2] GlcNAc epimerase, [3] N-acetylglucosamine-6-phosphate-N-acetyltransferase, [4] CMP-NeuAc synthase, and [5] sialyltransferase may be present separately in any combination of 1 to 4 types, preferably 1 to 3 types, more preferably 1 or 2 types, and most preferably 1 type of recombinant DNA.

### «[1] NeuAc synthase»

The NeuAc synthase refers to a protein having a NeuAc synthase activity. The NeuAc synthase activity refers to an activity of producing NeuAc using ManNAc and PEP as substrates.

The NeuAc synthase is not particularly limited as long as it has a NeuAc synthase activity, and examples thereof include NeuAc synthase CjneuB of Campylobacter jejuni strain ATCC 43438, and proteins described in WO2015/037698.

The target protein can be confirmed to have the NeuAc synthase activity by preparing a recombinant DNA having a DNA encoding the protein, culturing a microorganism obtained by transforming a microorganism having no NeuAc synthase activity, for example, Escherichia coli strain W3110 with the recombinant DNA, preparing a cell extract containing the protein from the obtained culture product, bringing the fraction into contact with ManNAc and PEP as substrates, and detecting NeuAc produced as a result by high performance chromatography or gas chromatography.

Examples of the microorganism for producing the NeuAc synthase include a microorganism obtained by transforming a parent strain with a recombinant DNA containing a DNA encoding NeuAc synthase and having an enhanced NeuAc synthase activity as compared with the parent strain.

Examples of the DNA encoding NeuAc synthase include a DNA encoding CjneuB that is preferably derived from prokaryotes such as bacteria or from yeasts, particularly preferably derived from prokaryotes, and most preferably derived from Campylobacter jejuni strain ATCC 43438 (SEQ ID NO: 7).

The microorganism having an enhanced NeuAc synthase activity as compared with the parent strain refers to a microorganism in which the transcription amount of the DNA or the production amount of a protein encoded by the DNA is increased by introducing the recombinant DNA into the parent strain as an autonomously replicable plasmid, or by incorporating the recombinant DNA into the chromosome of the parent strain.

As a method for confirming that the transcription amount of the above DNA or the production amount of the protein encoded by the DNA is increased, for example, it can be confirmed by measuring the transcription amount of the DNA by Northern blotting or the production amount of the protein by Western blotting, and comparing the transcription amount of the DNA or the production amount of the protein with that of a parent strain.

### «(2) GlcNAc epimerase»

The GlcNAc epimerase is a protein having a GlcNAc epimerase activity. The GlcNAc epimerase activity refers to an activity of producing ManNAc by an epimerization reaction using GlcNAc as a substrate.

Examples of a microorganism for producing GlcNAc epimerase include a microorganism belonging to the genus Synechocystis, and a microorganism obtained by transforming a parent strain with a recombinant DNA having a DNA encoding GlcNAc epimerase and having an enhanced GlcNAc epimerase activity as compared with the parent strain.

Preferred examples of the microorganism belonging to the genus Synechocystis include Synechocystis sp. strain PCC 6803 (Pasteur Culture Collection of Cyanobacteria, INSTITUTE PASTEUR).

Examples of the DNA encoding GlcNAc epimerase include a DNA encoding GlcNAc epimerase that is preferably derived from prokaryotes such as bacteria or from yeasts, particularly preferably derived from prokaryotes, and most preferably derived from Synechocystis sp. strain PCC 6803 (SEQ ID NO: 8).

For example, a DNA encoding GlcNAc epimerase derived from Synechocystis sp. strain PCC 6803 can be obtained by the method described in WO00/47730.

By transforming the parent strain with a recombinant DNA having a DNA encoding GlcNAc epimerase, the microorganism having an enhanced GlcNAc epimerase activity as compared with the parent strain can be constructed. The microorganism can be confirmed to be a microorganism having an enhanced GlcNAc epimerase activity as compared with the parent strain by comparing the transcription amount of the DNA encoding GlcNAc epimerase and the production amount of the protein with those of the parent strain according to the method described above. Examples of such a microorganism include W3110/pRcneuB2.

### <<(3) N-acetylglucosamine-6-phosphate-N-acetyltransferase>>

The N-acetylglucosamine-6-phosphate-N-acetyltransferase refers to a protein having a N-acetylglucosamine-6-phosphate-N-acetyltransferase activity. The N-acetylglucosamine-6-phosphate-N-acetyltransferase activity refers to an activity of producing N-acetyl-D-glucosamine-6-phosphate from acetyl-CoA and D-glucosamine-6-phosphate.

Examples of the microorganism for producing N-acetylglucosamine-6-phosphate-N-acetyltransferase include a microorganism belonging to the genus Saccharomyces, and a microorganism obtained by transforming a parent strain with a recombinant DNA having a DNA encoding N-acetylglucosamine-6-phosphate-N-acetyltransferase and having an enhanced N-acetylglucosamine-6-phosphate-N-acetyltransferase activity as compared with the parent strain.

Preferred examples of the microorganism belonging to the genus Saccharomyces include Saccharomyces cerevisiae strain S288C.

Examples of the DNA encoding N-acetylglucosamine-6-phosphate-N-acetyltransferase include a DNA encoding N-acetylglucosamine-6-phosphate-N-acetyltransferase that is preferably derived from prokaryotes such as bacteria or from yeasts, particularly preferably derived from yeasts, and most preferably derived from Saccharomyces cerevisiae strain S288C (SEQ ID NO: 9).

By transforming the parent strain with the recombinant DNA having a DNA encoding N-acetylglucosamine-6-phosphate-N-acetyltransferase, a microorganism having an enhanced N-acetylglucosamine-6-phosphate-N-acetyltransferase activity as compared with the parent strain can be constructed. The microorganism can be confirmed to be a microorganism having an enhanced N-acetylglucosamine-6-phosphate-N-acetyltransferase activity as compared with the parent strain, by comparing the transcription amount of the DNA encoding N-acetylglucosamine-6-phosphate-N-acetyltransferase and the production amount of the protein with those of the parent strain.

### «[4] CMP-NeuAc synthase»

The CMP-NeuAc synthase refers to a protein having a CMP-NeuAc synthase activity. The CMP-NeuAc synthase activity refers to an activity of producing CMP-NeuAc using CTP and NeuAc as substrates.

Examples of the microorganism for producing CMP-NeuAc synthase include a microorganism belonging to the genus Pasteurella and a microorganism obtained by transforming a parent strain with a recombinant DNA having a DNA encoding CMP-NeuAc synthase and having an enhanced CMP-NeuAc synthase activity as compared with the parent strain.

Preferred examples of the microorganism belonging to the genus Pasteurella include the Pasteurella multocida strain PM70.

The DNA encoding CMP-NeuAc synthase is preferably a DNA (JP2008-5794A) encoding CMP-NeuAc synthase that is preferably derived from prokaryotes such as bacteria or from yeasts, particularly preferably from prokaryotes, and most preferably from the Pasteurella multocida strain PM70.

For example, a DNA encoding CMP-NeuAc synthase derived from the Pasteurella multocida strain PM70 can be obtained by a method described in JP2008-5794A.

By transforming the parent strain with the recombinant DNA having a DNA encoding CMP-NeuAc synthase, a microorganism having an enhanced CMP-NeuAc synthase activity as compared with the parent strain can be constructed. The microorganism can be confirmed to be a microorganism having an enhanced CMP-NeuAc synthase activity as compared with the parent strain, by comparing the transcription amount of the DNA encoding CMP-NeuAc synthase and the production amount of the protein with those of the parent strain. Examples of such a microorganism include Escherichia coli N18-14/pMnK1 (JP2008-5794A).

### <<[5] Sialyltransferase>>

The sialyltransferase refers to a protein having a sialyltransferase activity. The sialyltransferase activity refers to an activity of producing a NeuAc-containing carbohydrate using CMP-NeuAc and a receptor carbohydrate as substrates.

Examples of the receptor carbohydrate as a substrate of the sialyltransferase include oligosaccharides, polysaccharides, and complex carbohydrates such as glycoproteins and glycolipids. Examples of oligosaccharides or polysaccharides as a substrate of the sialyltransferase include oligosaccharides or polysaccharides having galactose at a non-reducing terminal, or oligosaccharides or polysaccharides having NeuAc at a non-reducing terminal.

Among the above oligosaccharides and polysaccharides, preferred is an oligosaccharide having a structure selected from the group consisting of lactose, globotriose, N-acetyllactosamine, lacto-N-tetraose, lacto-N-neotetraose, Lewis a, and Lewis X at a non-reducing terminal, or an oligosaccharide having a structure selected from the group consisting of NeuAcα2-Galβ1-4Glc and NeuAcα2-3Galβ1-4GlcNAc at a non-reducing terminal, and more preferred is lactose.

Examples of the complex carbohydrate as a substrate of the sialyltransferase include a complex carbohydrate in which a protein, a lipid, or the like is bound to the above oligosaccharide and polysaccharide.

Examples of the NeuAc-containing carbohydrate include a carbohydrate obtained by adding NeuAc to the above receptor carbohydrate. Preferred examples thereof include carbohydrates containing an oligosaccharide having a structure selected from the group consisting of NeuAcα2-3Galβ1-4Glc, NeuAcα2-6Galβ1-4Glc, and NeuAcα2-8NeuAc at a non-reducing terminal. More preferred examples thereof include 3'-sialyllactose (3' SL), 6'-sialyllactose (6' SL), sialyllacto-N-tetraose a (LST-a), sialyllacto-N-tetraose b (LST-b), sialyllacto-N-tetraose c (LST-c), and disialyllacto-N-tetraose (DSLNT).

Specific examples of the sialyltransferase include well-known enzymes such as α2,3-sialyltransferase for producing 3'-sialyllactose using CMP-NeuAc and lactose as substrates, and α2,6-sialyltransferase for producing 6'-sialyllactose using CMP-NeuAc and lactose as substrates.

Examples of the microorganism for producing sialyltransferase include a microorganism belonging to the genus Pasteurella, a microorganism belonging to the genus Photobacterium, and a microorganism obtained by transforming a parent strain with a recombinant DNA having a DNA encoding sialyltransferase and having an enhanced sialyltransferase activity as compared with the parent strain.

Preferred examples of the microorganism belonging to the genus Pasteurella include the Pasteurella multocida strain PM70. Preferred examples of the microorganism belonging to the genus Photobacterium include Photobacterium damselae strain JT0160.

Examples of the DNA encoding sialyltransferase include a DNA (WO03/27297) encoding α-2,3-sialyltransferase that is preferably derived from prokaryotes such as bacteria or from yeasts, particularly preferably derived from prokaryotes, and most preferably derived from Pasteurella multocida strain PM70, or a DNA (GenBank ACCESSION No.BAA25316) encoding α-2,6-sialyltransferase derived from Photobacterium damselae strain JT0160.

For example, the DNA encoding α-2,3-sialyltransferase derived from Pasteurella multocida strain PM70 and the DNA encoding α-2,6-sialyltransferase derived from Photobacterium damselae strain JT0160 can be obtained by a method described in WO03/27297.

By transforming the parent strain with a recombinant DNA having a DNA encoding sialyltransferase, a microorganism having an enhanced sialyltransferase activity as compared with the parent strain can be constructed.

The microorganism constructed by the above method can be confirmed to be a microorganism having an enhanced sialyltransferase activity as compared with the parent strain, by comparing the transcription amount of a DNA encoding a protein having the sialyltransferase activity and the production amount of the protein with those of the parent strain. Examples of such a microorganism include Escherichia coli NM522/pYP3 (JP2008-5794A).

### <Microorganism 2 in which ability to produce NeuAc and/or NeuAc-containing carbohydrate is artificially imparted or enhanced>

Among the microorganisms in which the ability to produce NeuAc and/or a NeuAc-containing carbohydrate is artificially imparted or enhanced, the microorganisms obtained by the method (c) or (d) are specifically preferably, for example, microorganisms in which a transcription factor for negatively regulating an activity of a gene encoding an enzyme associated with NeuAc biosynthesis is lost, and microorganisms in which the NeuAc decomposition activity is reduced.

### <<Microorganisms whose transcription factor for negatively regulating activity of gene encoding enzyme associated with NeuAc biosynthesis is lost>>

Examples of the transcription factors for negatively regulating the activity of a gene encoding an enzyme associated with NeuAc biosynthesis include yhbJ, fruR, and era. From the viewpoint of specificity for NeuAc production, yhbJ is preferable among them.

yhbJ is a transcription control factor for negatively regulating an activity of the glmS gene encoding L-glutamine-D-fructose 6-phosphate aminotransferase associated with NeuAc biosynthesis.

Examples of the genetic modification that causes loss of a function of the transcription factor for negatively regulating the activity of the gene encoding an enzyme associated with NeuAc biosynthesis include reducing or completely stopping the function of a protein encoded by the DNA by adding a modification to a DNA encoding a portion corresponding to the protein whose function is to be lost among genomic DNAs of a host.

The form of the modification added to the above DNA is not particularly limited as long as it is a form in which the function of the protein encoded by the DNA encoding the portion corresponding to the protein whose function is to be lost is reduced or completely stopped, and a well-known method can be appropriately used.

Examples of the form in which the function of the protein encoded by the portion corresponding to the protein whose function is to be lost is reduced or completely stopped include any one modification selected from the following (i) to (iii).
(i) All or a part of the DNA encoding the portion corresponding to the protein whose function is to be lost is removed.
(ii) The substitution, deletion, or addition of one or several bases is performed on the DNA encoding the portion corresponding to the protein whose function is to be lost.
(iii) The DNA encoding the portion corresponding to the protein whose function is to be lost is substituted with a DNA sequence whose identity with a DNA sequence before modification is less than 80%.

When the protein whose function is to be lost is yhbJ, as the loss of the function of yhbJ, the activity of yhbJ is, for example, preferably 20% or less, more preferably 10% or less, and still more preferably 5% or less, as compared with a non-modified strain.

The activity of yhbJ can be confirmed by examining an expression level of GlmS by a Western blot method or the like [Kalamorz F.et al., Mol Microbiol. 65(6): 1518-33 (2007)].

### <<Microorganism having reduced NeuAc decomposition activity>>

Examples of the microorganism having a reduced NeuAc decomposition activity include a microorganism having a reduced NeuAc lyase activity. Examples of the microorganism having a reduced NeuAc lyase activity include Escherichia coli NAN8-71 (FERM BP-7908, WO03/72783).

### 1-2. Microorganism whose enterobacterial common antigen (ECA) biosynthesis pathway is blocked

The microorganism according to the present invention is a microorganism which has an ability to produce N-acetylneuraminic acid or a N-acetylneuraminic acid-containing carbohydrate and whose ECA biosynthesis pathway is blocked. Examples of a method for blocking the ECA biosynthesis pathway include a method for causing loss of the activity of a protein associated with the ECA biosynthesis pathway.

Examples of the method for causing loss of the activity of the protein associated with the ECA biosynthesis pathway include a method for causing loss of a function of a specific protein in the same manner as the method described above in the items of <<Microorganisms whose transcription factor for negatively regulating activity of gene encoding enzyme associated with NeuAc biosynthesis is lost>>. Specific examples of the method include a method for knocking out a specific gene using PCR [Baba T. et al., Mol systems Biol (2006)], a method for using a homologous recombination system of a lambda phage [Proc. Natl. Acad. Sci. USA, 97, 6641-6645 (2000)], a method for inhibiting the expression of a specific gene by RNAi, and a method for introducing mutations into specific genes and promoter regions thereof using mutation agents such as nitrosoguanidine and ultraviolet light.

Examples of the protein associated with the ECA biosynthesis pathway include WecA, WecB, WecC, WecD, WecE, WecF, and WecG.

WecA refers to a protein having a UDP-N-acetylglucosamine-undecaprenyl phosphate N-acetylglucosamine phosphotransferase activity associated with ECA biosynthesis.

Examples of WecA include WecA that is preferably derived from prokaryotes such as bacteria or from yeasts, more preferably derived from prokaryotes, particularly preferably derived from Escherichia coli, and most preferably derived from Escherichia coli strain BW25113 (SEQ ID NO: 2).

Examples of a DNA encoding WecA include a DNA encoding WecA that is preferably derived from prokaryotes such as bacteria or from yeasts, more preferably derived from prokaryotes, particularly preferably derived from Escherichia coli, and most preferably derived from Escherichia coli strain BW25113 (SEQ ID NO: 1).

WecB refers to a protein having a UDP-N-acetylglucosamine 2-epimerase activity associated with ECA biosynthesis. Examples of WecB include WecB that is preferably derived from prokaryotes such as bacteria or from yeasts, more preferably derived from prokaryotes, particularly preferably derived from Escherichia coli, and most preferably derived from Escherichia coli strain BW25113 (SEQ ID NO: 4).

Examples of a DNA encoding WecB include a DNA encoding WecB that is preferably derived from prokaryotes such as bacteria or from yeasts, more preferably derived from prokaryotes, particularly preferably derived from Escherichia coli, and most preferably derived from Escherichia coli strain BW25113 (SEQ ID NO: 3).

WecC refers to a protein having a UDP-N-acetylmannosamine dehydrogenase activity associated with ECA biosynthesis. Examples of WecC include WecC that is preferably derived from prokaryotes such as bacteria or from yeasts, more preferably derived from prokaryotes, particularly preferably derived from Escherichia coli, and most preferably derived from Escherichia coli strain BW25113 (SEQ ID NO: 6).

Examples of a DNA encoding WecC include a DNA encoding WecC that is preferably derived from prokaryotes such as bacteria or from yeasts, more preferably derived from prokaryotes, particularly preferably derived from Escherichia coli, and most preferably derived from Escherichia coli strain BW25113 (SEQ ID NO: 5).

WecD refers to a protein having a dTDP-4-amino-4,6-dideoxy-D-galactose acyltransferase activity associated with ECA biosynthesis. Examples of WecD include WecD that is preferably derived from prokaryotes such as bacteria or from yeasts, more preferably derived from prokaryotes, particularly preferably derived from Escherichia coli, and most preferably derived from Escherichia coli strain BW25113 (SEQ ID NO: 52).

Examples of a DNA encoding WecD include a DNA encoding WecD that is preferably derived from prokaryotes such as bacteria or from yeasts, more preferably derived from prokaryotes, particularly preferably derived from Escherichia coli, and most preferably derived from Escherichia coli strain BW25113 (SEQ ID NO: 51).

WecE refers to a protein having a dTDP-4-dehydro-6-deoxy-D-glucose transaminase activity associated with ECA biosynthesis. Examples of WecE include WecE that is preferably derived from prokaryotes such as bacteria or from yeasts, more preferably derived from prokaryotes, particularly preferably derived from Escherichia coli, and most preferably derived from Escherichia coli strain BW25113 (SEQ ID NO: 54).

Examples of a DNA encoding WecE include a DNA encoding WecE that is preferably derived from prokaryotes such as bacteria or from yeasts, more preferably derived from prokaryotes, particularly preferably derived from Escherichia coli, and most preferably derived from Escherichia coli strain BW25113 (SEQ ID NO: 53).

WecF is a protein having a dTDP-N-acetylfucosamine: lipid II N-acetylfucosaminyltransferase activity associated with ECA biosynthesis. Examples of WecF include WecF that is preferably derived from prokaryotes such as bacteria or from yeasts, more preferably derived from prokaryotes, particularly preferably derived from Escherichia coli, and most preferably derived from Escherichia coli strain BW25113 (SEQ ID NO: 56).

Examples of a DNA encoding WecF include a DNA that is preferably derived from prokaryotes such as bacteria or from yeasts, more preferably derived from prokaryotes, particularly preferably derived from Escherichia coli, and most preferably derived from Escherichia coli strain BW25113 (SEQ ID NO: 55).

WecG refers to a protein having a UDP-N-acetyl-D-mannosaminouronate transferase activity associated with ECA biosynthesis. Examples of WecG include WecG that is preferably derived from prokaryotes such as bacteria or from yeasts, more preferably derived from prokaryotes, particularly preferably derived from Escherichia coli, and most preferably derived from Escherichia coli strain BW25113 (SEQ ID NO: 58).

Examples of a DNA encoding WecG include a DNA encoding WecG that is preferably derived from prokaryotes such as bacteria or from yeasts, more preferably derived from prokaryotes, particularly preferably derived from Escherichia coli, and most preferably derived from Escherichia coli strain BW25113 (SEQ ID NO: 57).

As the microorganism whose ECA biosynthesis pathway is blocked, specifically, a microorganism in which an activity of at least one protein selected from the following [1] to [3] is lost is preferable.
[1] A protein consisting of an amino acid sequence represented by SEQ ID NO: 2 or a homologous sequence thereof and having a WecA activity
[2] A protein consisting of an amino acid sequence represented by SEQ ID NO: 4 or a homologous sequence thereof, and having a WecB activity
[3] A protein consisting of an amino acid sequence represented by SEQ ID NO: 6 or a homologous sequence thereof and having a WecC activity

The identity of a nucleotide sequence or an amino acid sequence can be determined using an algorithm BLAST (Pro. Nat. Acad. Sci. USA, 90, 5873, 1993) or FASTA (Methods Enzymol., 183, 63, 1990) developed by Karlin and Altschul. Programs called BLASTN and BLASTX have been developed based on the algorithm BLAST (J. Mol. Biol., 215, 403, 1990). When a nucleotide sequence is analyzed using BLASTN based on BLAST, parameters are, for example, Score = 100 and word length = 12. When an amino acid sequence is analyzed using BLASTX based on BLAST, parameters are, for example, score = 50 and word length = 3. When the BLAST program and the Gapped BLAST program are used, default parameters for each program are used. A specific method of the analysis methods is well known.

The identity between the target amino acid sequence (amino acid sequence represented by SEQ ID NO: 2, 4, or 6) and the homologous sequence thereof is preferably 80% or more, more preferably 90% or more, still more preferably 95% or more, particularly preferably 98% or more, and most preferably 99% or more.

A method for obtaining a protein having a homologous sequence (hereinafter, also referred to as homologous protein) is not particularly limited, and a well-known method can be used. Specific examples thereof include the following method. First, nucleotide sequences having an identity of preferably 80% or more, more preferably 90% or more, still more preferably 95% or more, and most preferably 99% or more with the nucleotide sequence encoding the target amino acid sequence are searched for in various gene sequence databases. Subsequently, using a probe DNA or a primer DNA that can be designed based on the nucleotide sequence or the amino acid sequence obtained by the search, and a microorganism having the DNA, the homologous protein can be obtained by Southern hybridization against a chromosomal DNA library of the microorganism, preferably the genus Escherichia, more preferably Escherichia coli, and still more preferably Escherichia coli strain BW25113 using a probe that can be designed based on the nucleotide sequence of the DNA, and by PCR [PCR Protocols, Academic press (1990)] using, as a template, a primer DNA that can be designed based on the DNA encoding the target protein and using the chromosomal DNA of the microorganism, preferably the genus Escherichia, more preferably Escherichia coli, and still more preferably Escherichia coli strain BW25113.

The homologous sequence may be an amino acid sequence obtained by artificially deleting or substituting an amino acid residue from an original amino acid sequence or artificially inserting or adding an amino acid residue into the original amino acid sequence. The expression "an amino acid is deleted, substituted, inserted, or added in the homologous protein" may mean that 1 to 20 amino acids are deleted, substituted, inserted, or added at any position in the same sequence.

The alignment of the amino acid sequences can be created by using, for example, a well-known alignment program ClustalW [Nucelic Acids Research 22, 4673 (1994)]. For example, a default value can be used for parameters when creating an alignment using ClustalW.

When an amino acid is substituted, inserted, or added to a target amino acid sequence, the amino acid residue to be substituted, inserted, or added may be natural or non-natural. Examples of the natural amino acid include L-alanine, L-alanine, L-asparagine, L-aspartic acid, L-glutamine, L-glutamic acid, glycine, L-histidine, L-isoleucine, L-leucine, L-lysine, L-arginine, L-methionine, L-phenylalanine, L-proline, L-serine, L-threonine, L-tryptophan, L-tyrosine, L-valine, and L-cysteine.

Examples of mutually substitutable amino acids are shown below. Amino acids contained in the same group can be mutually substituted.
Group A: leucine, isoleucine, norleucine, valine, norvaline, alanine, 2-aminobutanoic acid, methionine, o-methylserine, t-butylglycine, t-butylalanine, and cyclohexylalanine
Group B: aspartic acid, glutamic acid, isoaspartic acid, isoglutamic acid, 2-aminoadipic acid, and 2-aminosuberic acid
Group C: asparagine and glutamine
Group D: lysine, arginine, omithine, 2,4-diaminobutanoic acid, and 2,3-diaminopropionic acid
Group E: proline, 3-hydroxyproline, and 4-hydroxyproline
Group F: serine, threonine, and homoserine
Group G: phenylalanine and tyrosine

The activity of the protein associated with the ECA biosynthesis pathway can be confirmed by, for example, examining the expression level of the ECA gene by a Northern blot method, a Western blot method, or the like. Specifically, for example, the WecA activity, WecB activity, or WecC activity of homologous proteins can be confirmed by the following methods.

The homologous protein consisting of the homologous sequence of the amino acid sequence represented by SEQ ID NO: 2 can be confirmed to have the WecA activity by, for example, the following method. First, a recombinant DNA having a DNA encoding a protein whose activity is to be confirmed is prepared. Next, a microorganism obtained by transforming a microorganism having no WecA activity, for example, Escherichia coli strain BW25113 ΔyhbJ ΔwecA described below with the recombinant DNA is cultured, and a cell extract containing the homologous protein is prepared from the obtained culture product. The cell extract containing the homologous protein is brought into contact with an aqueous solution containing undecaprenyl phosfate and UDP-N-acetylglucosamine as substrates to produce Lipid I in the aqueous solution. Finally, the homologous protein can be confirmed to have the WecA activity by detecting Lipid I in the reaction solution using an analyzer SPD-20A (manufactured by Shimadzu Corporation) described below.

The homologous protein consisting of the homologous sequence of the amino acid sequence represented by SEQ ID NO: 4 can be confirmed to have the WecB activity by, for example, the following method. First, a recombinant DNA having a DNA encoding a protein whose activity is to be confirmed is prepared. Next, a microorganism obtained by transforming a microorganism having no WecB activity, for example, Escherichia coli strain BW25113 ΔyhbJ ΔwecB described below with the recombinant DNA is cultured, and a cell extract containing the homologous protein is prepared from the obtained culture product. The cell extract containing the homologous protein is brought into contact with an aqueous solution containing UDP-N-acetylglucosamine as a substrate to produce UDP-N-acetylmannosamine in the aqueous solution. Finally, the homologous protein can be confirmed to have the WecB activity by detecting UDP-N-acetylmannosamine in the reaction solution using an analyzer SPD-20A (manufactured by Shimadzu Corporation) described below.

The homologous protein consisting of the homologous sequence of the amino acid sequence represented by SEQ ID NO: 6 can be confirmed to have the WecC activity by, for example, the following method. First, a recombinant DNA having a DNA encoding a protein whose activity is to be confirmed is prepared. Next, a microorganism obtained by transforming a microorganism having no WecC activity, for example, Escherichia coli strain BW25113 ΔyhbJ ΔwecC described below with the recombinant DNA is cultured, and a cell extract containing the homologous protein is prepared from the obtained culture product. The cell extract containing the homologous protein is brought into contact with an aqueous solution containing UDP-N-acetylmannosamine as a substrate to produce UDP-ManNAcA in the aqueous solution. Finally, the homologous protein can be confirmed to have the WecC activity by detecting UDP-N-acetyl-D-mannosaminouronate in the reaction solution using an analyzer SPD-20A (manufactured by Shimadzu Corporation) described below.

The DNA encoding the protein consisting of the amino acid sequence represented by SEQ ID NO: 2, 4, or 6 can be obtained by, for example, Southern hybridization against a chromosomal DNA library of the microorganism, preferably the genus Escherichia, more preferably Escherichia coli, and still more preferably Escherichia coli strain BW25113 using a probe that can be designed based on the nucleotide sequence of the DNA encoding the amino acid sequence represented by SEQ ID NO: 2, 4, or 6, and by PCR [PCR Protocols, Academic press (1990)] using a primer DNA that can be designed based on the DNA encoding the amino acid sequence represented by SEQ ID NO: 2, 4, or 6 and using, as a template, the chromosomal DNA of the microorganism, preferably the genus Escherichia, more preferably Escherichia coli, and still more preferably Escherichia coli strain BW25113.

Specific examples of the DNA encoding the amino acid sequence represented by SEQ ID NO: 2 include a DNA having the nucleotide sequence represented by SEQ ID NO: 1. Specific examples of the DNA encoding the amino acid sequence represented by SEQ ID NO: 4 include a DNA having the nucleotide sequence represented by SEQ ID NO: 3. Specific examples of the DNA encoding the amino acid sequence represented by SEQ ID NO: 6 include a DNA having the nucleotide sequence represented by SEQ ID NO: 5.

The loss of the activity of the protein associated with the ECA biosynthesis pathway in the microorganism obtained by the method described above can be confirmed by, for example, examining the expression level of the gene encoding the protein associated with the ECA biosynthesis pathway described above by a Northern blot method, a Western blot method, or the like.

The microorganisms constructed by the method described above can be confirmed to have an improved production efficiency of NeuAc and/or a NeuAc-containing carbohydrate compared with the parent strain by, for example, the following method. First, the parent strain and the constructed microorganisms are cultured in a culture medium. Subsequently, NeuAc and/or the NeuAc-containing carbohydrate accumulated in the culture product is detected using HPLC or a carbohydrate analyzer described below, and the production amount of the parent strain is compared with that of the constructed microorganism, thereby confirming that the production efficiency of the NeuAc and/or the NeuAc-containing carbohydrate is improved as compared with the parent strain.

### 2. Method for Producing NeuAc and/or NeuAc-containing Carbohydrate Using Microorganism

A method for producing NeuAc and/or a NeuAc-containing carbohydrate according to the present invention is characterized in that the above-described microorganism is cultured in a culture medium to produce NeuAc and/or the NeuAc-containing carbohydrate in the culture product.

The method for culturing a microorganism can be performed according to a usual method used for culturing a microorganism.

As a culture medium for culturing a microorganism, any of a natural culture medium and a synthetic culture medium may be used as long as the culture medium contains a carbon source, a nitrogen source, an inorganic salt, and the like that can be assimilated by the microorganism and can efficiently culture the transformant.

Any carbon source may be used as long as it can be assimilated by the microorganism, and examples thereof include saccharides such as glucose, fructose, sucrose, molasses containing these, starch, or a starch hydrolysate, organic acids such as acetic acid or propionic acid, or alcohols such as glycerol, ethanol, or propanol.

Examples of the nitrogen source include ammonium salts of inorganic acids or organic acids such as ammonia, ammonium chloride, ammonium sulfate, ammonium acetate, or ammonium phosphate, other nitrogen-containing compounds thereof, peptone, a meat extract, a yeast extract, a corn steep liquor, a casein hydrolyzate, a soybean meal, a soybean meal hydrolyzate, various fermented bacterial cells, and digestive products thereof.

Examples of the inorganic salt include potassium primary phosphate, potassium secondary phosphate, magnesium phosphate, magnesium sulfate, sodium chloride, ferrous sulfate, manganese sulfate, copper sulfate, and calcium carbonate.

The culture is generally preferably performed under preferred conditions such as shaking culture or deep aeration stirring culture. The culture temperature is generally 15°C to 40°C, and the culture time is generally 5 hours to 7 days. The pH of the culture solution during culture is generally maintained at 3.0 to 9.0. The pH is adjusted using an inorganic or organic acid, an alkaline solution, urea, calcium carbonate, ammonia, or the like.

If necessary, an antibiotic such as ampicillin or tetracycline may be added to the culture medium. When culturing a microorganism transformed with an expression vector using an inducible promoter as a promoter, an inducer may be added to the culture medium as necessary. For example, isopropyl-β-D-thiogalactopyranoside (IPTG) or the like may be added to the culture medium when culturing a microorganism transformed with an expression vector using a lac promoter, and indole acrylic acid or the like may be added to the culture medium when culturing a microorganism transformed with an expression vector using a trp promoter.

In the case of producing the NeuAc-containing carbohydrate, the culture medium preferably contains a receptor carbohydrate such as lactose. The receptor carbohydrate may be added at the beginning of the culture, or may be added as necessary during the culture. A concentration of the added receptor carbohydrate in the culture medium is preferably 10 g/l to 300 g/l.

In the method for producing the NeuAc-containing carbohydrate, when the microorganism to be used does not have the ability to produce a receptor carbohydrate such as lactose, instead of adding a receptor carbohydrate such as lactose to the culture medium during culture, a receptor carbohydrate such as lactose may be supplied to the transformant according to the present invention by culturing microorganisms having the ability to produce a receptor carbohydrate such as lactose from a saccharide simultaneously with the microorganism according to the present invention.

NeuAc or the NeuAc-containing carbohydrate can be produced by generating and accumulating NeuAc or the NeuAc-containing carbohydrate in the culture product by the above culture, and collecting NeuAc or the NeuAc-containing carbohydrate from the culture product.

The collection of NeuAc or a NeuAc-containing carbohydrate from the above culture product can be generally carried out by combining an activated carbon method, an ion exchange resin method, a precipitation method, and other well-known methods. When NeuAc or a NeuAc-containing carbohydrate is accumulated in bacterial cells, NeuAc or the NeuAc-containing carbohydrate can be collected, by using an ion exchange resin method or the like, from a supernatant obtained by, for example, crushing the bacterial cells with ultrasonic waves or the like and removing the bacterial cells by centrifugation.

NeuAc or the NeuAc-containing carbohydrate can be quantified using a carbohydrate analyzer manufactured by Dionex Corporation, a two-wavelength absorbance detector manufactured by Shimadzu Corporation, or the like [Anal. Biochem., 189, 151 (1990)]. Examples

### [Analysis Example]

In Examples, analysis and quantification of NeuAc were performed by the following procedure. A culture solution containing microorganisms after culture was centrifuged, and the supernatant was collected. NeuAc contained in the supernatant was analyzed by an analyzer SPD-20A (manufactured by Shimadzu Corporation).

### [Analysis Conditions]

Column: Shodex RSpak KC-811
Column temperature: 40°C
Eluent composition: 1% phosphoric acid aqueous solution
Flow rate: 0.5 ml/min
Detector: SPD-20A

### [Example 1] Production of Microorganisms Used for Production of NeuAc

### (1) Construction of NeuAc Production Plasmid

A plasmid expressing NeuAc synthase gene CjneuB (SEQ ID NO: 7) derived from Campylobacter jejuni strain ATCC 43438, N-GluNAc 2-epimerase gene slr1975 (SEQ ID NO: 8) derived from Synechocystis sp. strain PCC 6803, and N-acetylglucosamine-6-phosphate-N-acetyltransferase gene GNA1 (SEQ ID NO: 9) derived from Saccharomyces cerevisiae strain S288C was prepared by the following procedure.

The Campylobacter jejuni strain ATCC 43438, the Synechocystis sp. strain PCC 6803, or the Saccharomyces cerevisiae strain S288C was cultured by a known culture method, and the chromosomal DNA of each microorganism was isolated and purified. Using the prepared DNA, PCR was performed using, as a primer set, DNAs consisting of the nucleotide sequences shown in "Primer set" in Table 1 and using, as a template, a DNA described in "Template" in Table 1 to obtain each amplified DNA fragment.

**[Table 1]**

| Primer set (SEQ ID NO:) | Template | Amplified DNA fragment |
|---|---|---|
| 10 and 11 | Genomic DNA of Campylobacter jejuni ATCC 43438 strain | CjneuB |
| 12 and 13 | DNA derived from Synechocystis sp. PCC 6803 | slrl975 |
| 14 and 15 | DNA derived from Saccharomyces cerevisiae S288C | GNA1 |

PCR was performed using, as a template, a mixture of the CjneuB fragment, the slr1975 fragment, and the GNA1 fragment obtained as described above at an equimolar ratio and using, as a primer set, DNAs consisting of the nucleotide sequences represented by SEQ ID NOs: 10 and 15 to obtain a DNA fragment of about 2.7 kb in which the CjneuB fragment, the slr1975 fragment, and the GNA1 fragment were linked to each other (hereinafter, referred to as a CjneuB-slr1975-GNA1 fragment).

The CjneuB-slr1975-GNA1 fragment and an expression vector pTrc99a (manufactured by GE Healthcare Bioscience) were linked to each other using In-Fusion HD Cloning Kit (manufactured by Takara Bio Inc.) to obtain an expression plasmid pTrc99a-CjneuB-s1r1975-GNA1 (hereinafter referred to as pCSG1).

### (2) Construction of NeuAc Production Host

### <Escherichia Coli whose yhbJ Gene is lost>

Escherichia coli in which transcription regulation factor yhbJ gene (SEQ ID NO: 16) for negatively regulating an activity of a glmS gene encoding L-glutamine-D-fructose 6-phosphate aminotransferase associated with NeuAc biosynthesis was disrupted was prepared by the following procedure.

A BW25113 ΔyhbJ strain whose yhbJ gene was completely deleted was obtained by the same method as in Baba et al. [Baba T. et al. (2006) Mol systems Biol] using the BW25113 strain (Keio collection (Systematic single-gene knock-out mutants of E. coli K-12)) as a host.

### <Acquisition of DNA fragment to be used as marker for gene deletion>

PCR was performed using, as a primer set, DNAs consisting of the nucleotide sequences shown in "Primer set" in Table 2 and using, as a template, a DNA described in "Template" in Table 2 to obtain each amplified DNA fragment.

**[Table 2]**

| Primer set (SEQ ID NO:) | Template | Amplified DNA fragment |
|---|---|---|
| 17 and 18 | PIISG 396 (manufactured by Takara Bio Inc.) | cat |
| 19 and 20 | Genomic DNA of Bacillus subtilis 168 strain | sacB |

A genomic DNA of the Bacillus subtilis strain 168 was prepared by a standard method. The cat of the amplified DNA fragment contains about 200 bp upstream to about 50 bp downstream of the cat (chloramphenicol acetyltransferase) gene on pHSG396. The sacB of the amplified DNA fragment contains about 300 bp upstream to about 100 bp downstream of the sacB (levansucrase) gene on the genomic DNA of the Bacillus subtilis strain 168.

Next, PCR was performed using, as a template, a mixture of the cat and sacB of the amplified DNA fragment at an equimolar ratio and using, as a primer set, DNAs consisting of the nucleotide sequences represented by SEQ ID NOs: 17 and 20 to obtain a DNA fragment containing the cat gene and the sacB gene (hereinafter, referred to as cat-sacB).

### <Escherichia coli whose wecA gene is lost>

Escherichia coli in which wecA gene (SEQ ID NO: 1) encoding UDP-N-acetylglucosamine-undecaprenyl phosphate N-acetylglucosamine phosphotransferase associated with the ECA biosynthesis was disrupted was produced by the following procedure using, as a parent strain, the BW25113 ΔyhbJ strain constructed as described above.

PCR was performed using, as a template, the chromosomal DNA of the BW25113 strain prepared by a common method and using, as a primer set, DNAs consisting of the nucleotide sequences shown in "Primer set" in Table 3 to obtain each amplified DNA fragment.

**[Table 3]**

| Primer set (SEQ ID NO:) | Amplified DNA fragment | Remarks |
|---|---|---|
| 21 and 22 | wecA upstream 1 | Nucleotide sequence represented by SEQ ID NO: 17 is complementary to nucleotide sequence at 5' end of nucleotide sequence represented by SEQ ID NO: 22 |
| 23 and 24 | wecA downstream 1 | Nucleotide sequence represented by SEQ ID NO: 20 is complementary to nucleotide sequence at 5' end of nucleotide sequence represented by SEQ ID NO: 23 |
| 25 and 26 | wecA upstream 2 | Nucleotide sequence represented by SEQ ID NO: 26 is complementary to nucleotide sequence represented by SEQ ID NO: 27 |
| 27 and 28 | wecA downstream 2 | |

The wecA upstream 1 and the wecA upstream 2 contain about 1500 bp upstream from the initiation codon of the wecA gene. The wecA downstream 1 and the wecA downstream 2 contain about 1300 bp downstream from a termination codon of the wecA gene.

PCR was performed using, as a template, a mixture of the wecA upstream 1, the wecA downstream 1, and the cat-sacB fragment at an equimolar ratio and using, as a primer set, DNAs consisting of the nucleotide sequences represented by SEQ ID NOs: 29 and 30 to obtain a DNA fragment consisting of a sequence with the cat-sacB fragment inserted into a sequence in a region around the wecA gene (hereinafter referred to as wecA::cat-sacB).

PCR was performed using, as a template, a mixture of wecA upstream 2 and wecA downstream 2 at an equimolar ratio and using, as a primer set, DNAs consisting of the nucleotide sequences represented by SEQ ID NOs: 29 and 30 to obtain a DNA fragment consisting of a sequence with the wecA upstream and wecA downstream directly linked to each other without wecA (hereinafter referred to as ΔwecA).

The wecA::cat-sacB fragment was introduced into a BW25113 ΔyhbJ strain carrying a plasmid pKD46 containing a gene encoding λ recombinase [Datsenko, K. A., Warner, B. L., Proc. Natl. Acad. Sci., USA, Vol.97, 6640-6645 (2000)] by an electroporation method to obtain a transformant (a transformant with the wecA gene substituted with wecA::cat-sacB) exhibiting chloramphenicol resistance and sucrose sensitivity.

The ΔwecA fragment was introduced into the transformant by an electroporation method to obtain a transformant (a transformant with wecA::cat-sacB substituted with ΔwecA) exhibiting chloramphenicol sensitivity and sucrose resistance. The transformant was named BW25113 ΔyhbJ ΔwecA strain.

### <Escherichia coli whose wecB Gene is lost>

Escherichia coli in which wecB gene (SEQ ID NO: 3) encoding UDP-N-acetylglucosamine-2-epimerase associated with ECA biosynthesis was disrupted was prepared by the following procedure using the BW25113 ΔyhbJ strain as a parent strain.

PCR was performed using, as a template, the chromosomal DNA of the BW25113 strain prepared by a common method and using, as a primer set, DNAs consisting of the nucleotide sequences shown in "Primer set" in Table 4 to obtain each amplified DNA fragment.

**[Table 4]**

| Primer set (SEQ ID NO:) | Amplified DNA fragment | Remarks |
|---|---|---|
| 3 1 and 32 | wecB upstream 1 | Nucleotide sequence represented by SEQ ID NO: 17 is complementary to nucleotide sequence at 5' end of nucleotide sequence represented by SEQ ID NO: 30 |
| 33 and 34 | wecB downstream 1 | Nucleotide sequence represented by SEQ ID NO: 20 is complementary to nucleotide sequence at 5' end of nucleotide sequence represented by SEQ ID NO: 33 |
| 35 and 36 | wecB upstream 2 | Nucleotide sequence represented by SEQ ID NO: 36 is complementary to nucleotide sequence represented by SEQ ID NO: 37 |
| 37 and 38 | wecB downstream 2 | |

The wecB upstream 1 and the wecB upstream 2 contain about 1400 bp upstream from an initiation codon of the wecB gene. The wecB downstream 1 and the wecB downstream 2 contain about 1400 bp downstream from an initiation codon of the wecB gene.

PCR was performed using, as a template, a mixture of wecB upstream 1, wecB downstream 1, and a cat-sacB fragment at an equimolar ratio and using, as a primer set, DNAs consisting of the nucleotide sequences represented by SEQ ID NOs: 39 and 40 to obtain a DNA fragment consisting of a sequence with the cat-sacB fragment inserted into a sequence in a region around the wecB gene (hereinafter referred to as wecB::cat-sacB).

PCR was performed using, as a template, a mixture of wecB upstream 2 and wecB downstream 2 at an equimolar ratio and using, as a primer set, DNAs consisting of the nucleotide sequences represented by SEQ ID NOs: 39 and 40 to obtain a DNA fragment consisting of a sequence with the wecB upstream and the wecB downstream directly linked to each other without wecB (hereinafter referred to as ΔwecB).

The wecB::cat-sacB fragment was introduced into the BW25113 ΔyhbJ strain carrying a plasmid pKD46 containing a gene encoding λ recombinase by an electroporation method to obtain a transformant (a transformant with the wecB gene substituted with wecB: :cat-sacB) exhibiting chloramphenicol resistance and sucrose sensitivity.

The ΔwecB fragment was introduced into the transformant by the electroporation method to obtain a transformant (a transformant with wecB::cat-sacB substituted with ΔwecB) exhibiting chloramphenicol sensitivity and sucrose resistance. The transformant was named BW25113 ΔyhbJ ΔwecB strain.

### <Escherichia coli whose wecC Gene is lost>

Escherichia coli in which wecC gene (SEQ ID NO: 5) encoding UDP-N-acetylmannosamine dehydrogenase associated with ECA biosynthesis was disrupted was prepared using the BW25113 ΔyhbJ strain as a parent strain by the following procedure.

PCR was performed using, as a template, the chromosomal DNA of the BW25113 strain prepared by a common method and using, as a primer set, DNAs consisting of the nucleotide sequences shown in "Primer set" in Table 5 to obtain each amplified DNA fragment.

**[Table 5]**

| Primer set (SEQ ID NO:) | Amplified DNA fragment | Remarks |
|---|---|---|
| 4 1 and 42 | wecC upstream 1 | Nucleotide sequence represented by SEQ ID NO: 17 is complementary to nucleotide sequence at 5' end of nucleotide sequence represented by SEQ ID NO: 42 |
| 43 and 44 | wecC downstream 1 | Nucleotide sequence represented by SEQ ID NO: 20 is complementary to nucleotide sequence at 5' end of nucleotide sequence represented by SEQ ID NO: 43 |
| 45 and 46 | wecC upstream 2 | Nucleotide sequence represented by SEQ ID NO: 46 is complementary to nucleotide sequence represented by SEQ ID NO: 47 |
| 47 and 48 | wecC downstream 2 | |

The wecC upstream 1 and the wecC upstream 2 contain about 1400 bp upstream from an initiation codon of the wecC gene. The wecC downstream 1 and the wecC downstream 2 contain about 1400 bp downstream from a termination codon of the wecC gene.

PCR was performed using, as a template, a mixture of wecC upstream 1, wecC downstream 1, and a cat-sacB fragment at an equimolar ratio and using, as a primer set, DNAs consisting of the nucleotide sequences represented by SEQ ID NOs: 49 and 50 to obtain a DNA fragment consisting of a sequence with the cat-sacB fragment inserted into a sequence in a region around the wecC gene (hereinafter referred to as wecC::cat-sacB).

PCR was performed using, as a template, a mixture of wecC upstream 2 and wecC downstream 2 at an equimolar ratio and using, as a primer set, DNAs consisting of the nucleotide sequences represented by SEQ ID NOs: 49 and 50 to obtain a DNA fragment consisting of a sequence with the wecC upstream and the wecC downstream directly linked to each other without wecC (hereinafter referred to as ΔwecC).

The wecC::cat-sacB fragment was introduced into the BW25113 ΔyhbJ strain carrying a plasmid pKD46 containing a gene encoding λ recombinase by an electroporation method to obtain a transformant (a transformant with the wecC gene substituted with wecC::cat-sacB) exhibiting chloramphenicol resistance and sucrose sensitivity.

The ΔwecC fragment was introduced into the transformant by an electroporation method to obtain a transformant (a transformant with wecC::cat-sacB substituted with ΔwecC) exhibiting chloramphenicol sensitivity and sucrose resistance. The transformant was named BW25113 ΔyhbJ ΔwecC strain.

### (3) Construction of microorganism having NeuAc production plasmid

The plasmid pCSG1 obtained in the above (1) was separately transformed into the BW25113 ΔyhbJ strain, the BW25113 ΔyhbJ ΔwecA strain, the BW25113 ΔyhbJ ΔwecB strain, and the BW25113 ΔyhbJ ΔwecC strain constructed in the above (2). In order to perform the transformation, the microorganisms were selected with an LB agar culture medium containing 100 mg/L of ampicillin sodium according to a standard method. The strains obtained by the transformation were named BW25113 ΔyhbJ/pCSG1 strain, BW25113 ΔyhbJ ΔwecA/pCSG1 strain, BW25113 ΔyhbJ ΔwecB/pCSG1 strain, and BW25113 ΔyhbJ ΔwecC/pCSG1 strain, respectively.

### [Example 2] Production of NeuAc according to fermentation method

The BW25113 ΔyhbJ/pCSG1 strain, BW25113 ΔyhbJ ΔwecA/pCSG1 strain, BW25113 ΔyhbJ ΔwecB/pCSG1 strain, and BW25113 ΔyhbJ ΔwecC/pCSG1 strain obtained in Example 1 were cultured overnight on an LB plate containing 100 mg/L of ampicillin at 30°C, followed by inoculating into a large-sized test tube containing 5 ml of LB culture medium containing 100 mg/L of ampicillin and culturing at 30°C for 16 hours.

Thereafter, 0.3 ml of each culture solution was inoculated into a large-sized test tube containing 3 ml of a production culture medium [glucose 30 g/L, magnesium sulfate heptahydrate 2.0 g/L, dipotassium hydrogen phosphate 16 g/L, potassium dihydrogen phosphate 14 g/L, ammonium sulfate 2.0 g/L, citric acid monohydrate 1.0 g/L, casamino acid (manufactured by Difco Corporation) 5.0 g/L, vitamin B1 10 mg/L, iron sulfate heptahydrate 50 mg/L, and manganese sulfate heptahydrate 10 mg/L (adjusted to pH 7.2 by aqueous sodium hydroxide, and then autoclaved)], [aqueous solutions of glucose and magnesium sulfate heptahydrate were separately prepared, autoclaved, cooled, and then mixed] containing 100 mg/L of ampicillin, and then isopropyl-β-D-thiogalactopyranoside (IPTG) was added to reach a concentration of 0.125 mM, followed by shaking-culture at 30°C for 30 hours.

After completion of the culture, the culture solution was appropriately diluted, and a sample after the filter sterilization was subjected to HPLC analysis to quantify a concentration of NeuAc contained in a supernatant and an amount of NeuAc produced per bacterial cell. The results are shown in Table 6.

A ratio of NeuAc to all the peak compounds detected in the analysis, that is, the HPLC purity of NeuAc is shown in Table 7.

**[Table 6]**

| Strain | NeuAc [g/L] | NeuAc [g/L/OD] |
|---|---|---|
| BW25113 ΔyhbJ/pCSG1 strain | 0.20 | 0.023 |
| BW25113 ΔyhbJ ΔwecA/pCSG1 strain | 0.28 | 0.028 |
| BW25113 ΔyhbJ ΔwecB/pCSG1 strain | 0.25 | 0.027 |
| BW25113 ΔyhbJ ΔwecC/pCSG1 strain | 0.26 | 0.026 |

**[Table 7]**

| Strain | NeuAc% (relative to all peak compounds) |
|---|---|
| BW25113 ΔyhbJ/pCSG1 strain | 1.13 |
| BW25113 ΔyhbJ ΔwecA/pCSG1 strain | 1.58 |
| BW25113 ΔyhbJ ΔwecB/pCSG1 strain | 1.46 |
| BW25113 ΔyhbJ ΔwecC/pCSG1 strain | 1.42 |

As shown in Tables 6 and 7, it was revealed that the NeuAc-producing ability higher than that of the parent strain was exhibited by disrupting any one of the wecA gene, the wecB gene, and the wecC gene associated with ECA biosynthesis. In addition, the ratio of NeuAc to all products contained in the culture solution was improved, and therefore, it was shown that the reduction of impurities was also effective. From these results, it was found that, by using a microorganism which has an ability to produce NeuAc or a NeuAc-containing carbohydrate and whose ECA biosynthesis pathway is blocked, NeuAc or the NeuAc-containing carbohydrate can be produced more efficiently than in the related art.

Although the present invention has been described in detail with reference to specific embodiments, it will be apparent to those skilled in the art that various changes and modifications can be made without departing from the spirit and scope of the present invention. The present application is based on a Japanese Patent Application No. 2021-166476 filed on October 8, 2021, the entire contents of which are incorporated herein by reference. All references cited herein are incorporated in their entirety.

### SEQUENCE LISTING FREE TEXT

SEQ ID NO: 1: nucleotide sequence of wecA
SEQ ID NO: 2: amino acid sequence of WecA
SEQ ID NO: 3: nucleotide sequence of wecB
SEQ ID NO: 4: amino acid sequence of WecB
SEQ ID NO: 5: nucleotide sequence of wecC
SEQ ID NO: 6: amino acid sequence of WecC
SEQ ID NO: 7: nucleotide sequence of CjneuB
SEQ ID NO: 8: nucleotide sequence of slr1975
SEQ ID NO: 9: nucleotide sequence of GNA1
SEQ ID NO: 10: nucleotide sequence of a primer Fw for amplifying a CjneuB fragment
SEQ ID NO: 11: nucleotide sequence of a primer Rv for amplifying a CjneuB fragment
SEQ ID NO: 12: nucleotide sequence of a primer Fw for amplifying of a slr1975 fragment
SEQ ID NO: 13: nucleotide sequence of a primer Rv for amplifying a slr1975 fragment
SEQ ID NO: 14: nucleotide sequence of a primer Fw for amplifying a GNA1 fragment
SEQ ID NO: 15: nucleotide sequence of a primer Rv for amplifying a GNA1 fragment
SEQ ID NO: 16: nucleotide sequence of yhbJ
SEQ ID NO: 17: nucleotide sequence of a primer Fw for amplifying a cat fragment
SEQ ID NO: 18: nucleotide sequence of a primer Rv for amplifying a cat fragment
SEQ ID NO: 19: nucleotide sequence of a primer Fw for amplifying a sacB fragment
SEQ ID NO: 20: nucleotide sequence of a primer Rv for amplifying a sacB fragment
SEQ ID NO: 21: nucleotide sequence of a primer_Fw for amplifying wecA upstream 1 fragment
SEQ ID NO: 22: nucleotide sequence of a primer_Rv for amplifying wecA upstream 1 fragment
SEQ ID NO: 23: nucleotide sequence of a primer_Fw for amplifying wecA downstream 1 fragment
SEQ ID NO: 24: nucleotide sequence of a primer_Rv for amplifying wecA downstream 1 fragment
SEQ ID NO: 25: nucleotide sequence of a primer_Fw for amplifying wecA upstream 2 fragment
SEQ ID NO: 26: nucleotide sequence of a primer_Rv for amplifying wecA upstream 2 fragment
SEQ ID NO: 27: nucleotide sequence of a primer_Fw for amplifying wecA downstream 2 fragment
SEQ ID NO: 28: nucleotide sequence of a primer_Rv for amplifying wecA downstream 2 fragment
SEQ ID NO: 29: nucleotide sequence of a primer_Fw for producing fragment for wecA disruption
SEQ ID NO: 30: nucleotide sequence of a primer_Rv for producing fragment for wecA disruption
SEQ ID NO: 31: nucleotide sequence of a primer_Fw for amplifying wecB upstream 1 fragment
SEQ ID NO: 32: nucleotide sequence of a primer_Rv for amplifying wecB upstream 1 fragment
SEQ ID NO: 33: nucleotide sequence of a primer_Fw for amplifying wecB downstream 1 fragment
SEQ ID NO: 34: nucleotide sequence of a primer_Rv for amplifying wecB downstream 1 fragment
SEQ ID NO: 35: nucleotide sequence of a primer_Fw for amplifying wecB upstream 2 fragment
SEQ ID NO: 36: nucleotide sequence of a primer_Rv for amplifying wecB upstream 2 fragment
SEQ ID NO: 37: nucleotide sequence of a primer_Fw for amplifying wecB downstream 2 fragment
SEQ ID NO: 38: nucleotide sequence of a primer_Rv for amplifying wecB downstream 2 fragment
SEQ ID NO: 39: nucleotide sequence of a primer _Fw for producing fragment for wecB disruption
SEQ ID NO: 40: nucleotide sequence of a primer_Rv for producing fragment for wecB disruption
SEQ ID NO: 41: nucleotide sequence of a primer_Fw for amplifying wecC upstream 1 fragment
SEQ ID NO: 42: nucleotide sequence of a primer_Rv for amplifying wecC upstream 1 fragment
SEQ ID NO: 43: nucleotide sequence of a primer_Fw for amplifying wecC downstream 1 fragment
SEQ ID NO: 44: nucleotide sequence of a primer_Rv for amplifying wecC downstream 1 fragment
SEQ ID NO: 45: nucleotide sequence of a primer_Fw for amplifying wecC upstream 2 fragment
SEQ ID NO: 46: nucleotide sequence of a primer_Rv for amplifying wecC upstream 2 fragment
SEQ ID NO: 47: nucleotide sequence of a primer_Fw for amplifying wecC downstream 2 fragment
SEQ ID NO: 48: nucleotide sequence of a primer_Rv for amplifying wecC downstream 2 fragment
SEQ ID NO: 49: nucleotide sequence of a primer_Fw for producing fragment for wecC disruption
SEQ ID NO: 50: nucleotide sequence of a primer_Rv for producing fragment for wecC disruption
SEQ ID NO: 51: nucleotide sequence of wecD
SEQ ID NO: 52: amino acid sequence of WecD
SEQ ID NO: 53: nucleotide sequence of wecE
SEQ ID NO: 54: amino acid sequence of WecE
SEQ ID NO: 55: nucleotide sequence of wecF
SEQ ID NO: 56: amino acid sequence of WecF
SEQ ID NO: 57: nucleotide sequence of wecG
SEQ ID NO: 58: amino acid sequence of WecG

## Claims

1. A microorganism which has an ability to produce at least one of N-acetylneuraminic acid and a N-acetylneuraminic acid-containing carbohydrate and whose enterobacterial common antigen (ECA) biosynthesis pathway is blocked.

2. The microorganism according to claim 1, wherein an activity of at least one protein selected from the following [1] to [3] is lost,
[1] a protein consisting of an amino acid sequence represented by SEQ ID NO: 2 or a homologous sequence thereof and having a UDP-N-acetylglucosamine-undecaprenyl phosphate N-acetylglucosamine phosphotransferase (WecA) activity;
[2] a protein consisting of an amino acid sequence represented by SEQ ID NO: 4 or a homologous sequence thereof and having a UDP-N-acetylglucosamine 2-epimerase (WecB) activity; and
[3] a protein consisting of an amino acid sequence represented by SEQ ID NO: 6 or a homologous sequence thereof and having UDP-N-acetylmannosamine dehydrogenase (WecC) activity.

3. The microorganism according to claim 1 or 2, which is Escherichia coli.

4. A method for producing at least one of N-acetylneuraminic acid and a N-acetylneuraminic acid-containing carbohydrate, the method comprising:
culturing the microorganism according to any one of claims 1 to 3 in a culture medium to produce at least one of N-acetylneuraminic acid and the N-acetylneuraminic acid-containing carbohydrate.
